# FASCICULE DE BREVET EUROPEEN

(11) **EP 0 900 552 B1**
(45) Date de publication et mention de la délivrance du brevet: **04.12.2002**
(21) Numéro de dépôt: 98420151.7
(22) Date de dépôt: 08.09.1998
(51) Int. Cl.: A61F 2/34

(54) **Implant acétabulaire à bouchons**
Hüftgelenkpfannenimplantat mit Stopfen
Acetabular implant with plugs

(30) Priorité: 08.09.1997 FR 9711337
(43) Date de publication de la demande: 10.03.1999
(73) Titulaire: Tornier SA, 38330 Saint Ismier (FR)
(72) Inventeur: Tornier, Alain, 38330 Saint Ismier (FR)
(74) Mandataire: Schmitt, John

(56) Documents cités:
- EP-A- 0 346 270
- EP-A- 0 444 381
- EP-A- 0 677 280
- EP-A- 0 771 552
- WO-A-94/05234
- WO-A-95/15734
- DE-U- 29 516 473
- FR-A- 2 638 963
- US-A- 5 370 702
- US-A- 5 571 198
- US-A- 5 609 648

## Description

La présente invention est relative à un implant acétabulaire ou cotyloïdien destiné à être placé dans une cavité articulaire endommagée pour coopérer notamment avec la tête d'une prothèse fémorale, l'ensemble constituant une prothèse totale de hanche.

On connaît des implants acétabulaires réalisés entièrement en matière plastique telle que du polyéthylène qui sont destinés à coopérer avec une tête fémorale naturelle ou artificielle dans une arthroplastie de la hanche. De tels implants sont mis en place en utilisant un ciment qui solidarise lesdits implants avec la cavité osseuse endommagée.

On connaît également des implants acétabulaires comportant un cotyle métallique et un insert en matière plastique qui est logé dans la cavité interne dudit cotyle. Ce dernier peut être fixé à l'intérieur du cotyle humain d'un patient à l'aide, soit de ciment, soit directement par l'intermédiaire de vis qui pénètrent dans l'os iliaque.

Lorsque l'implant est cimenté ou vissé dans le cotyle humain d'un patient, la paroi extérieure du cotyle métallique est soit recouverte d'une épaisseur d'hydroxyapatite, soit pourvue d'un état de surface rugueux pour permettre la repousse osseuse et par conséquent la fixation de l'implant.

L'implant acétabulaire à vis comporte un cotyle métallique qui est percé sur sa périphérie d'un certain nombre de trous permettant au chirurgien plusieurs solutions pour la mise en place des vis de fixation.

Le nombre de vis et leur position sur la périphérie du cotyle métallique dépendent généralement de l'état osseux du cotyle humain afin que chaque vis réalise un ancrage parfait dans la direction qui a été déterminée par le chirurgien.

Lorsque tous les trous de vis ne sont pas utilisés par le chirurgien, les trous libres sont obturés par des bouchons vissés ou sertis, de sorte que d'éventuels débris de polyéthylène ne puissent s'échapper à l'interface osseuse. Un tel cotyle métallique est connu du US-A-5 571 198.

On remarque que dans de tels cas, l'assemblage du bouchon dans le trou de vis n'est pas étanche, ce qui, dans le cas de traitement de la surface externe du cotyle métallique par projection de poudre, entraîne la pollution de la zone de contact prévue pour l'appui d'une éventuelle tête de vis.

On constate par ailleurs que, toujours lorsque l'implant acétabulaire fait l'objet d'un revêtement par projection de poudre, ledit revêtement s'écaille lors du démontage des bouchons dans le patient, produisant ainsi un amas de débris dommageables à la fixation du cotyle.

L'implant acétabulaire ou cotyloïdien suivant la présente invention comprend un cotyle métallique qui est percé d'un certain nombre de trous débouchant qui sont obstrués par des bouchons qui peuvent, suivant le cas opératoire, être retirés pour libérer un ou plusieurs trous pour la mise en place de vis de fixation, tandis que chaque bouchon est constitué d'une partie cylindrique filetée qui se prolonge par une partie sensiblement conique ou sphérique se terminant par un prolongement cylindrique permettant, lors du retrait du bouchon de découper suivant le contour du trou correspondant, une couche de revêtement dont est recouverte la face externe du cotyle métallique.

L'implant acétabulaire ou cotyloïdien suivant la présente invention comprend une assise conique ou sphérique du bouchon qui présente respectivement un angle ou un rayon de courbure inférieur à la cavité correspondante du cotyle métallique, afin d'assurer une surface de contact annulaire située du côté libre du bouchon de nature à assurer l'étanchéité de la liaison bouchon dans le cotyle métallique.

L'implant acétabulaire ou cotyloïdien suivant la présente invention comprend un prolongement qui présente un diamètre inférieur au diamètre débouchant des trous du cotyle métallique, préservant un jeu minimum qui est deux fois l'épaisseur du revêtement susceptible de garantir le découpage net d'une couche de revêtement selon le pourtour du trou.

L'implant acétabulaire ou cotyloïdien suivant la présente invention comprend un cotyle métallique qui comporte une cavité interne propre à recevoir l'insert en matière plastique, ladite cavité étant constituée du côté du bord libre dudit cotyle d'une portion de cône de faible conicité qui se prolonge par une autre portion à profil conique de forte conicité se terminant par un fond plat qui est percé en son milieu d'un trou débouchant fileté, tandis que la portion de cône de forte conicité est percée de plusieurs trous débouchant obstrués par des bouchons.

L'implant acétabulaire ou cotyloïdien suivant la présente invention comprend des trous qui comportent chacun du côté de la cavité interne une partie cylindrique filetée qui se prolonge en direction de l'extérieur du cotyle par un logement à profil sensiblement conique ou en creux.

L'implant acétabulaire ou cotyloïdien suivant la présente invention comprend des bouchons qui sont constitués chacun d'une partie cylindrique filetée qui se prolonge par une partie sensiblement conique ou sphérique afin de coopérer avec le profil du logement prévu dans chaque trou.

L'implant acétabulaire ou cotyloïdien suivant la présente invention comprend un cotyle métallique qui comporte une face extérieure qui est recouverte d'une couche permettant de rendre ladite face externe uniforme de manière que les trous et les bouchons ne soient pas visibles de l'extérieur dudit cotyle.

L'implant acétabulaire ou cotyloïdien suivant la présente invention comprend un trou débouchant central qui est obstrué par un bouchon.

L'implant acétabulaire ou cotyloïdien selon la présente invention prévoit un mode de réalisation de la partie concave des trous de l'implant acétabulaire et de la partie bombée des bouchons assurant une parfaite étanchéité empêchant toute pollution de la surface concave d'appui des vis.

L'implant acétabulaire ou cotyloïdien conforme à l'invention comporte un cotyle métallique dont la face extérieure présente un aspect uniforme et sensiblement rugueux pour permettre la fixation de l'implant dans le cotyle humain.

L'implant acétabulaire ou cotyloïdien selon la présent invention prévoit en outre une forme de bouchon présentant un embout tel que le démontage du bouchon n'entraîne pas d'écaillage de la couche de dépôt présente sur la périphérie du cotyle métallique.

Le cotyle métallique pour implant acétabulaire ou cotyloïdien suivant la présente invention, est percé d'un certain nombre de trous débouchant qui sont obstrués par des bouchons qui peuvent, suivant le cas opératoire, être retirés pour libérer un ou plusieurs trous pour la mise en place de vis de fixation, tandis que chaque bouchon est constitué d'une partie cylindrique filetée qui se prolonge par une partie sensiblement conique ou sphérique se terminant par un prolongement cylindrique permettant, lors du retrait du bouchon de découper suivant le contour du trou correspondant à la couche d'hydroxyapatite dont est recouverte la face externe du cotyle métallique.

Le cotyle métallique pour implant acétabulaire ou cotyloïdien suivant la présente invention comporte une cavité interne propre à recevoir l'insert en matière plastique, ladite cavité étant constituée du côté du bord libre dudit cotyle d'une portion de cône à faible conicité de faible conicité se prolongeant par une autre portion à profil conique de forte conicité se terminant par un fond plat qui est percé en son milieu d'un trou débouchant fileté, tandis que la portion de cylindre de forte conicité est percée de plusieurs trous débouchant obstrués par des bouchons.

La description qui va suivre en regard des dessins annexés, donnés à titre d'exemple non limitatif, permettra de mieux comprendre l'invention, les caractéristiques qu'elle présente et les avantages qu'elle est susceptible de procurer:
Figure 1 est une vue en perspective illustrant le cotyle métallique de l'implant acétabulaire ou cotyloïdien suivant la présente invention.
Figure 2 est une vue de dessus montrant le cotyle métallique suivant la présente invention.
Figure 3 est une coupe représentant le cotyle métallique sans bouchons.
Figure 4 est une coupe agrandie illustrant le bouchon qui est vissé dans chaque trou prévu sur la périphérie du cotyle métallique de l'implant suivant la présente invention.
Figure 5 est une coupe agrandie montrant un bouchon qui est vissé dans le trou central du cotyle métallique de l'implant suivant la présente invention.
Figure 6 est une coupe semblable à celle de figure 3, mais représentant les trous du cotyle métallique coopérant avec les bouchons.
Figure 7 est une vue illustrant en détail le bouchon à l'intérieur d'un trou du cotyle.

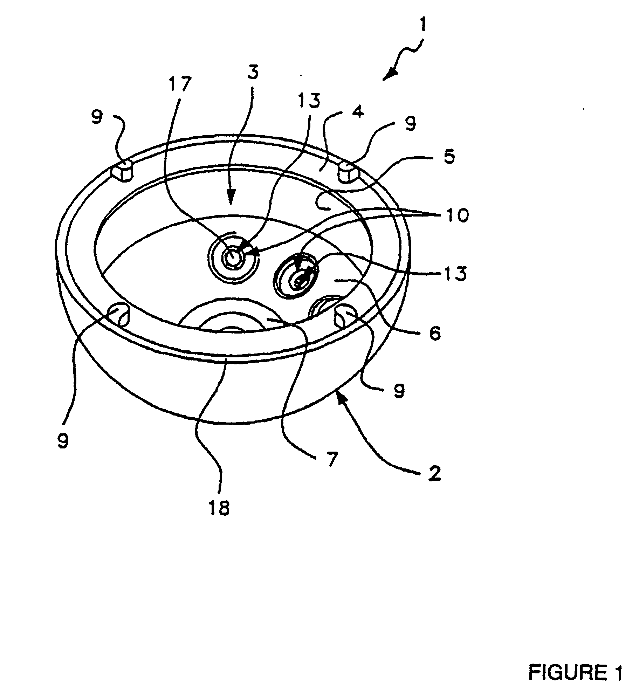

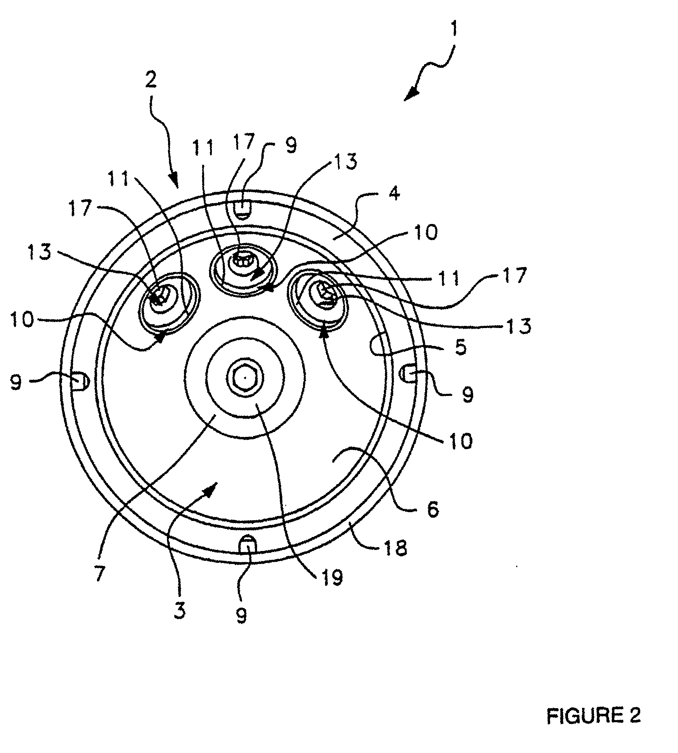

On a montré en figures 1, 2 et 3 un implant acétabulaire ou cotyloïdien 1 comportant un cotyle ou une calotte métallique 2 dans lequel s'engage un insert en matière plastique non représenté.

Le cotyle métallique 2 présente un profil extérieur hémisphérique destiné à venir se fixer dans une cavité ou cotyle humain d'un patient qui est préalablement aménagé.

Le cotyle métallique 2 peut être réalisé en alliage de titane allié ou en tout autre matériau biocompatible.

L'insert intérieur non représenté destiné à recevoir la boule d'un élément fémoral ou huméral peur être réalisé en une matière plastique biocompatible telle qu'un polyéthylène haute densité ou toute autre matière adaptée.

Le cotyle métallique 2 comporte une cavité interne 3 propre à recevoir l'insert en matière plastique. La cavité 3 est constituée du côté du bord libre 4 du cotyle 2 d'une portion de cône à faible conicité 5 qui se prolonge par une autre portion à profil conique 6 de forte conicité.

La portion à profil conique 6 se termine par un fond plat 7 qui est percé en son milieu d'un trou débouchant 8 comportant une partie filetée.

Il va de soi que le profil interne de la cavité 3 peut être différent sans pour autant changer l'objet de la présente invention.

Le bord libre 4 est solidaire d'ergots 9 régulièrement répartis sur le pourtour du cotyle métallique 2 pour permettre l'indexation angulaire de l'insert par rapport audit cotyle.

La cavité interne 3 est percée de plusieurs trous débouchant 10 permettant la mise en place de vis de fixation non représentées. Dans notre exemple, les trous 10 sont percés sur un même rayon disposé dans la portion à profil conique 6 de la cavité interne 3.

Chaque trou 10 comporte du côté de la cavité interne 3 une partie cylindrique 11 filetée qui se prolonge en direction de l'extérieur du cotyle 2 par un logement 12 à profil sensiblement conique ou en creux.

Le profil interne du logement 12 permet de régler la position angulaire de l'axe de la vis de fixation et de recevoir le profil externe de sa tête pour que cette demière soit le plus possible éloignée de la cavité 3 pour ne pas venir en contact avec l'insert en matière plastique.

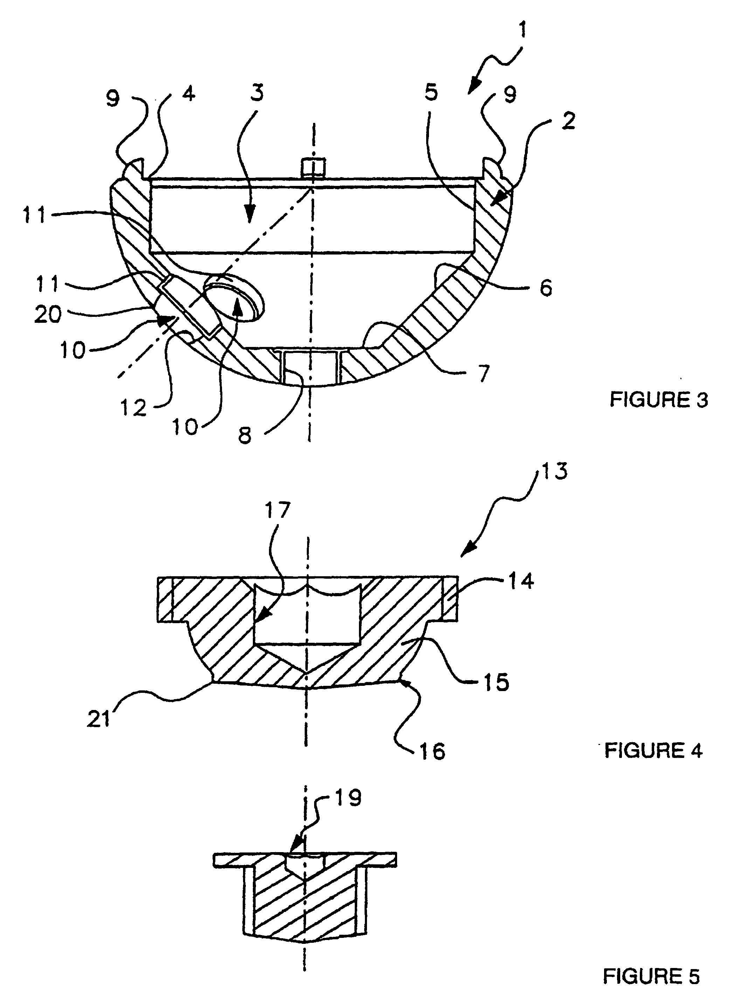

On note qu'à la fin de la fabrication, et après contrôle du cotyle métallique 2, que les trous 10 sont obstrués par des bouchons 13 comme montré en figure 4.

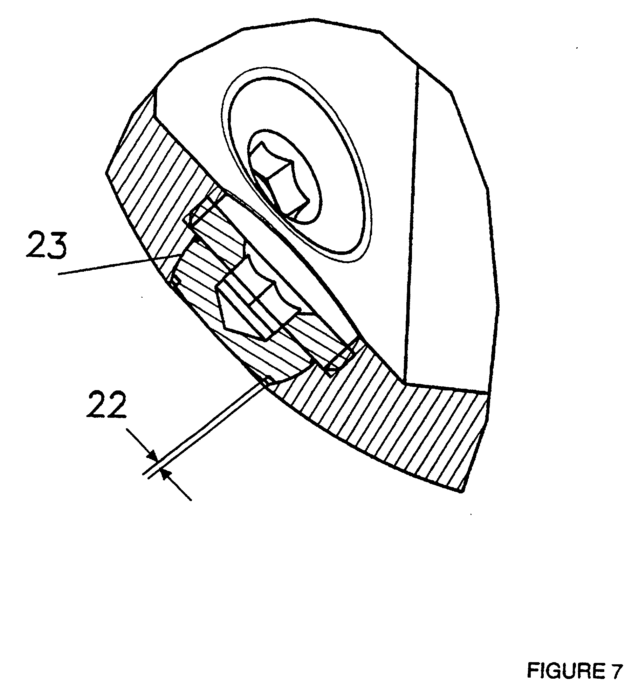

Chaque bouchon 13 est constitué d'une partie cylindrique filetée 14 qui se prolonge par une partie conique ou bombée ou sphérique 15. Cette dernière présente généralement un profil complémentaire à celui prévu pour le logement 12 de chaque trou 10. L'angle ou le rayon de courbure de la partie conique ou bombée 15 du bouchon 13 est réalisé avec une valeur plus faible que l'angle ou le rayon de courbure de la partie conique ou bombée 12 du cotyle 2, de sorte que la surface de contact 23 entre le bouchon 13 et la cavité 12 du cotyle 2 soit obligatoirement un anneau localisé du côté de l'extrémité libre du bouchon 13 (figure 7).

Chaque bouchon 13 est percé en son milieu, du côté de la partie 14, d'un trou borgne 17 formant une empreinte hexagonale destinée à recevoir une clef permettant la mise en place ou le retrait du bouchon.

Après la mise en place et le blocage des bouchons 13 dans chaque trou 10, la face externe du cotyle métallique 2 peut être recouverte d'une couche de dépôt d'hydroxyapatite 18 permettant la repousse osseuse et par conséquent la fixation secondaire de l'implant 1 dans le cotyle humain. On notera que grâce à la surface de contact 23 décrite plus haut entre le bouchon 13 et la cavité 12 du cotyle 2, la poudre d'hydroxyapatite constitutrice du revêtement 18 ne peut pas pénétrer et polluer la surface du logement 12.

Cette couche de dépôt 18 permet également de rendre la face externe du cotyle métallique 2 uniforme, c'est à dire, que les trous 10 et les bouchons 13 ne sont pas visibles de l'extérieur dudit cotyle.

La partie bombée 15 se termine par un prolongement cylindrique 16 de très faible longueur qui permet de prolonger le profil extérieur du cotyle au niveau de chaque trou 10 et de retenir au niveau desdits trous 10 la couche d'hydroxyapatite 18.

Le diamètre 21 du prolongement 16 des bouchons 13 est sensiblement plus faible que le diamètre 20 débouchant des cavités 12 du cotyle 2, de manière à préserver un espace 22 d'une valeur minimum de deux fois l'épaisseur du revêtement 18 (par exemple 0.3mm) propre à faciliter le découpage de la couche de dépôt 18 suivant le contour du trou 10 lors du démontage du bouchon 13.

Ainsi, le cotyle métallique 2 tel que décrit précédemment peur être utilisé par le chirurgien de plusieurs manières suivant le cas opératoire.

En premier lieu, le cotyle métallique 2 peut être impacté sans vis dans le cotyle humain d'un patient. En effet, sa face externe uniforme et sans trou est propre à coopérer parfaitement avec la cavité osseuse pour la fixation du cotyle.

En second lieu, le cotyle métallique 2 peut être ancré dans le cotyle humain à l'aide d'une ou plusieurs vis. Le chirurgien doit simplement procéder au retrait des bouchons 13 qu'il estime nécessaire à la fixation du cotyle 2. Lorsque les bouchons 13 sont retirés, les trous 10 sont conformés pour recevoir la tête de la vis et permettre un réglage angulaire de quelques degrés de l'axe longitudinal du corps de la vis.

On remarque que dans tous les cas le trou 8 prévu dans le fond 7 du cotyle 2 permet, d'une part la mise en place d'un ancillaire pour l'introduction dudit cotyle métallique dans celui humain, et d'autre part, après retrait de l'ancillaire, la visualisation du fond du cotyle humain pour renseigner le chirurgien sur la bonne position du cotyle métallique 2.

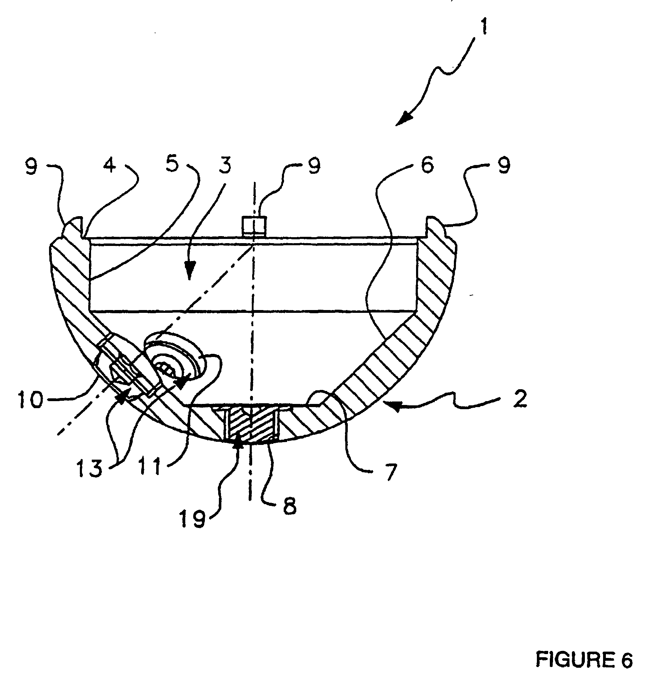

Lorsque le cotyle métallique 2 est en place dans celui humain, le chirurgien introduit dans le trou 8 un bouchon 19 ou un autre permettant de l'obstruer (figures 5 et 6).

On constate que dans tous les cas de mise en place du cotyle métallique 2 dans le cotyle humain, il n'y a jamais de trous 10 et 8 libres, c'est à dire, sans un bouchon ou une vis de fixation ce qui permet d'éviter le passage éventuelles particules provenant de l'usure de l'insert en matière plastique.

## Revendications

1. Implant acétabulaire ou cotyloïdien destiné à être placé dans une cavité ou cotyle articulaire humain endommagé, ledit implant étant constitué d'un cotyle métallique dans lequel est retenu un insert en matière plastique, le cotyle métallique (2) est percé d'un certain nombre de trous débouchant (10) qui sont obstrués par des bouchons (13) qui peuvent, suivant le cas opératoire, être retirés pour libérer un ou plusieurs trous (10) pour la mise en place de vis de fixation, tandis que chaque bouchon (13) est constitué d'une partie cylindrique filetée (14) qui se prolonge par une partie sensiblement conique ou sphérique (15), **caractérisé en ce que** le bouchon se termine par un prolongement cylindrique (16) permettant, lors du retrait du bouchon (13) de découper suivant le contour du trou (10) correspondant, une couche de revêtement (18) dont est recouverte la face externe du cotyle métallique (2).

2. Implant acétabulaire ou cotyloïdien suivant la revendication 1, **caractérisé en ce que** l'assise conique ou sphérique (15) du bouchon (13) présente respectivement un angle ou un rayon de courbure inférieur à la cavité (12) correspondante du cotyle métallique (2), afin d'assurer une surface de contact annulaire située du côté libre du bouchon (13) de nature à assurer l'étanchéité de la liaison bouchon (13) dans le cotyle métallique (2).

3. Implant acétabulaire ou cotyloïdien suivant la revendication 1, **caractérisé en ce que** le prolongement (16) présente un diamètre (21) qui est inférieur au diamètre débouchant (20) des trous (10) du cotyle métallique (2), préservant un jeu minimum qui est deux fois l'épaisseur du revêtement (18) susceptible de garantir le découpage net d'une couche de revêtement (18) selon le pourtour du trou (10).

4. implant acétabulaire ou cotyloïdien suivant la revendication 1, **caractérisé en ce que** le cotyle métallique (2) comporte une cavité interne (3) propre à recevoir l'insert en matière plastique, ladite cavité (3) étant constituée du côté du bord libre (4) dudit cotyle (2) d'une portion de cône de faible conicité (5) qui se prolonge par une autre portion à profil conique (6) de forte conicité se terminant par un fond plat (7) qui est percé en son milieu d'un trou débouchant fileté (8), tandis que la portion de cône (6) de forte conicité est percée de plusieurs trous débouchant (10) obstrués par des bouchons (13).

5. Implant acétabulaire ou cotyloïdien suivant la revendication 4, **caractérisé en ce que** chaque trou (10) comporte du côté de la cavité interne (3) une partie cylindrique filetée (11) qui se prolonge en direction de l'extérieur du cotyle (2) par un logement (12) à profil sensiblement conique ou en creux.

6. Implant acétabulaire ou cotyloïdien suivant la revendication 4, **caractérisé en ce que** chaque bouchon (13) est constitué d'une partie cylindrique filetée (14) qui se prolonge par une partie sensiblement conique ou sphérique (15) afin de coopérer avec le profil du logement (12) prévu dans chaque trou (10).

7. Implant acétabulaire ou cotyloïdien suivant la revendication 4, **caractérisé en ce que** le cotyle métallique (2) comporte une face extérieure qui est recouverte d'une couche (18) permettant de rendre ladite face externe uniforme de manière que les trous (10) et les bouchons (13) ne soient pas visibles de l'extérieur dudit cotyle.

8. Implant acétabulaire ou cotyloïdien suivant la revendication 6, **caractérisé en ce que** le trou débouchant central (8) est obstrué par un bouchon (19).

9. Cotyle métallique pour implant acétabulaire ou cotyloïdien suivant l'une quelconque des revendications précédentes, percé d'un certain nombre de trous débouchant (10) qui sont obstrués par des bouchons (13) qui peuvent, suivant le cas opératoire, être retirés pour libérer un ou plusieurs trous (10) pour la mise en place de vis de fixation, tandis que chaque bouchon (13) est constitué d'une partie cylindrique filetée (14) qui se prolonge par une partie sensiblement conique ou sphérique (15), **caractérisé en ce que** le bouchon se termine par un prolongement cylindrique (16) permettant, lors du retrait du bouchon (13) de découper suivant le contour du trou (10) correspondant à la couche d'hydroxyapatite (18) dont est recouverte la face externe du cotyle métallique (2).

10. Cotyle métallique suivant la revendication 9, **caractérisé en ce qu'**il comporte une cavité interne (3) propre à recevoir l'insert en matière plastique, ladite cavité (3) étant constituée du côté du bord libre (4) dudit cotyle (2) d'une portion de cône à faible conicité (5) de faible conicité se prolongeant par une autre portion à profil conique (6) de forte conicité se terminant par un fond plat (7) qui est percé en son milieu d'un trou débouchant fileté (8), tandis que la portion de cylindre (6) de forte conicité est percée de plusieurs trous débouchant (10) obstrués par des bouchons (13).

## Claims

1. Acetabular or cotyloid implant intended to be placed in a damaged human articular cavity or cotyle, said implant consisting of a metal cup in which a plastic insert is retained, the metal cup (2) being provided with a number of through-holes (10) which are stopped by plugs (13) which, depending on the operation, can be removed in order to free one or more holes (10) for placement of fixation screws, while each plug (13) is formed by a threaded cylindrical part (14) which is continued by a substantially conical or spherical part (15), **characterized in that** the plug ends in a cylindrical continuation (16) making it possible, upon removal of the plug (13), to cut off, along the contour of the corresponding hole (10), a layer of coating (18) with which the outer face of the metal cup (2) is covered.

2. Acetabular or cotyloid implant according to Claim 1, **characterized in that** the conical or spherical seat (15) of the plug (13) has, respectively, an angle or a radius of curvature less than the corresponding cavity (12) of the metal cup (2) in order to ensure an annular contact surface situated on the free side of the plug (13) of a nature to ensure the leaktightness of the plug connection (13) in the metal cup (2).

3. Acetabular or cotyloid implant according to Claim 1, **characterized in that** the continuation (16) has a diameter (21) which is less than the opening diameter (20) of the holes (10) of the metal cup (2), preserving a minimum play which is twice the thickness of the coating (18) capable of guaranteeing the clean cutting of a layer of coating (18) along the perimeter of the hole (10).

4. Acetabular or cotyloid implant according to Claim 1, **characterized in that** the metal cup (2) comprises an inner cavity (3) able to receive the plastic insert, said cavity (3) being formed, towards the free edge (4) of said cup (2), by a cone portion of low conicity (5) which is continued by another portion of conical profile (6) of high conicity ending in a flat base (7) which is drilled at its centre with a threaded through-hole (8), while the cone portion (6) of high conicity is drilled with several through-holes (10) stopped by plugs (13).

5. Acetabular or cotyloid implant according to Claim 4, **characterized in that** each hole (10) comprises, on the side towards the inner cavity (3), a threaded cylindrical part (11) which is continued in the direction of the outside of the cup (2) by a recess (12) of substantially conical or hollowed profile.

6. Acetabular or cotyloid implant according to Claim 4, **characterized in that** each plug (13) is formed by a threaded cylindrical part (14) which is continued by a substantially conical or spherical part (15) in order to cooperate with the profile of the recess (12) provided in each hole (10).

7. Acetabular or cotyloid implant according to Claim 4, **characterized in that** the metal cup (2) comprises an outer face which is covered with a layer (18) making it possible to render said outer face uniform so that the holes (10) and the plugs (13) are not visible from the outside of said cup.

8. Acetabular or cotyloid implant according to Claim 6, **characterized in that** the central through-hole (8) is stopped by a plug (19).

9. Metal cup for an acetabular or cotyloid implant according to any one of the preceding claims, drilled with a number of through-holes (10) which are stopped by plugs (13) which, depending on the operation, can be removed in order to free one or more holes (10) for placement of fixation screws, while each plug (13) is formed by a threaded cylindrical part (14) which is continued by a substantially conical or spherical part (15), **characterized in that** the plug ends in a cylindrical continuation (16) making it possible, upon removal of the plug (13), to cut off along the contour of the hole (10) corresponding to the layer of hydroxyapatite (18) with which the outer face of the metal cup (2) is covered.

10. Metal cup according to Claim 9, **characterized in that** it comprises an inner cavity (3) able to receive the plastic insert, said cavity (3) being formed, towards the free edge (4) of said cup (2), by a cone portion of low conicity (5) which is continued by another portion of conical profile (6) of high conicity ending in a flat base (7) which is drilled at its centre with a threaded through-hole (8), while the cylinder portion (6) of high conicity is drilled with several through-holes (10) stopped by plugs (13).

## Patentansprüche

1. Hüftgelenkpfannenimplantat oder Gelenkpfannenimplantat, das dazu bestimmt ist, in einen Hohlraum oder eine beschädigte menschliche Gelenkpfanne eingesetzt zu werden, wobei das Implantat aus einer metallischen Gelenkpfanne besteht, in der ein Einsatz aus Kunststoffmaterial gehalten wird, wobei die metallische Gelenkpfanne (2) von einer bestimmten Zahl von Durchgangslöchern (10) durchstoßen wird, die durch Stopfen (13) verschlossen sind, die je nach Operationsfall herausgenommen werden können, um ein Loch oder mehrere Löcher (10) für die Platzierung von Befestigungsschrauben freizulegen, wobei jeder Stopfen (13) aus einem mit einem Gewinde versehenen, zylindrischen Teil (14) besteht, der sich in einem im Wesentlichen konischen oder sphärischen Teil (15) fortsetzt, **dadurch gekennzeichnet, dass** der Stopfen in einem zylindrischen Fortsatz (16) endet, der es gestattet, bei der Herausnahme des Stopfens (13) eine Überzugschicht (18), mit der die Außenseite der metallischen Gelenkpfanne (2) bedeckt ist, entsprechend dem Umriss des entsprechenden Lochs (10) zu zerschneiden.

2. Hüftgelenkpfannenimplantat oder Gelenkpfannenimplantat nach Anspruch 1, **dadurch gekennzeichnet, dass** die konische oder sphärische Basis (15) des Stopfens (13) einen Winkel beziehungsweise einen Krümmungsradius aufweist, der kleiner als der entsprechende Hohlraum (12) der metallischen Gelenkpfanne (2) ist, um für eine ringförmige Kontaktfläche zu sorgen, die auf der freien Seite des Stopfens (13) liegt und geeignet ist, die Dichtheit der Stopfenverbindung (13) in der metallischen Gelenkpfanne (2) sicherzustellen.

3. Hüftgelenkpfannenimplantat oder Gelenkpfannenimplantat nach Anspruch 1, **dadurch gekennzeichnet, dass** der Fortsatz (16) einen Durchmesser (21) aufweist, der kleiner als der Mündungsdurchmesser (20) der Löcher (10) der metallischen Gelenkpfanne (2) ist, wodurch ein minimales Spiel beibehalten wird, das das Zweifache der Dicke des Überzugs (18) beträgt und in der Lage ist, den scharfen Schnitt einer Überzugsschicht (18) entlang dem Umfang des Lochs (10) zu gewährleisten.

4. Hüftgelenkpfannenimplantat oder Gelenkpfannenimplantat nach Anspruch 1, **dadurch gekennzeichnet, dass** die metallische Gelenkpfanne (2) einen inneren Hohlraum (3) umfasst, der geeignet ist, den Einsatz aus Kunststoffmaterial aufzunehmen, wobei der Hohlraum (3) auf der Seite des freien Randes (4) der Gelenkpfanne (2) aus einem konusförmigen Teil (5) mit geringer Konizität besteht, der sich in einem weiteren Teil mit konischem Profil (6) mit starker Konizität fortsetzt, welcher in einem flachen Boden (7) endet, der in seiner Mitte von einem mit einem Gewinde versehenen Durchgangsloch (8) durchstoßen wird, während der Konusteil (6) mit starker Konizität von mehreren Durchgangslöchern (10) durchstoßen wird, die durch Stopfen (13) verschlossen sind.

5. Hüftgelenkpfannenimplantat oder Gelenkpfannenimplantat nach Anspruch 4, **dadurch gekennzeichnet, dass** jedes Loch (10) auf der Seite des inneren Hohlraums (3) einen mit einem Gewinde versehenen, zylindrischen Teil (11) umfasst, der sich in Richtung zur Außenseite der Gelenkpfanne (2) hin in einer Aufnahme (12) mit im Wesentlichem konischen Profil oder einer vertieften Aufnahme fortsetzt.

6. Hüftgelenkpfannenimplantat oder Gelenkpfannenimplantat nach Anspruch 4, **dadurch gekennzeichnet, dass** jeder Stopfen (13) aus einem mit einem Gewinde versehenen, zylindrischen Teil (14) besteht, der sich in einem im Wesentlichen konischen oder sphärischen Teil (15) fortsetzt, um mit dem Profil der Aufnahme (12) zusammenzuwirken, die in jedem Loch (10) vorgesehen ist.

7. Hüftgelenkpfannenimplantat oder Gelenkpfannenimplantat nach Anspruch 4, **dadurch gekennzeichnet, dass** die metallische Gelenkpfanne (2) eine Außenfläche umfasst, die mit einer Schicht (18) bedeckt ist, die es gestattet, die Außenfläche gleichförmig zu machen, so dass die Löcher (10) und die Stopfen (13) von außerhalb der Gelenkpfanne nicht sichtbar sind.

8. Hüftgelenkpfannenimplantat oder Gelenkpfannenimplantat nach Anspruch 6, **dadurch gekennzeichnet, dass** das mittlere Durchgangsloch (8) durch einen Stopfen (19) verschlossen ist.

9. Metallische Gelenkpfanne für ein Hüftgelenkpfannenimplantat oder Gelenkpfannenimplantat nach einem der vorhergehenden Ansprüche, die von einer bestimmten Zahl von Durchgangslöchern (10) durchstoßen wird, die durch Stopfen (13) verschlossen sind, die je nach Operationsfall herausgenommen werden können, um ein Loch oder mehrere Löcher (10) für die Platzierung von Befestigungsschrauben freizulegen, wobei jeder Stopfen (13) aus einem mit einem Gewinde versehenen, zylindrischen Teil (14) besteht, der sich in einem im Wesentlichen konischen oder sphärischen Teil (15) fortsetzt, **dadurch gekennzeichnet, dass** der Stopfen in einem zylindrischen Fortsatz (16) endet, der es gestattet, bei der Herausnahme des Stopfens (13) eine Hydroxidapatitschicht (18), mit der die Außenseite der metallischen Gelenkpfanne (2) bedeckt ist, entsprechend dem Umriss des entsprechenden Lochs (10) zu zerschneiden.

10. Metallische Gelenkpfanne nach Anspruch 9, **dadurch gekennzeichnet, dass** sie einen inneren Hohlraum (3) umfasst, der geeignet ist, den Einsatz aus Kunststoffmaterial aufzunehmen, wobei der Hohlraum (3) auf der Seite des freien Randes (4) der Gelenkpfanne (2) aus einem konusförmigen Teil (5) mit geringer Konizität besteht, der sich in einem weiteren Teil mit konischem Profil (6) mit starker Konizität fortsetzt, welcher in einem flachen Boden (7) endet, der in seiner Mitte von einem mit einem Gewinde versehenen Durchgangsloch (8) durchstoßen wird, während der Konusteil (6) mit starker Konizität von mehreren Durchgangslöchern (10) durchstoßen wird, die durch Stopfen (13) verschlossen sind.
